(19) Europäisches Patentamt / European Patent Office / Office européen des brevets

(11) **EP 4 718 101 A1**

(12) **EUROPEAN PATENT APPLICATION**

(43) Date of publication:
**01.04.2026 Bulletin 2026/14**

(21) Application number: **24203534.3**

(22) Date of filing: **30.09.2024**

(51) International Patent Classification (IPC):
**G01R 33/56** (2006.01)  **G01R 33/561** (2006.01)
**A61B 6/03** (2006.01)  **G01R 33/563** (2006.01)

(52) Cooperative Patent Classification (CPC):
**G01R 33/561; G01R 33/5608;** A61B 6/032;
A61B 6/037; G01R 33/56341

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicants:
- **Centre Hospitalier Universitaire Vaudois**
  **1011 Lausanne (CH)**
- **Siemens Healthineers AG**
  **91301 Forchheim (DE)**

(72) Inventors:
- **Patiño-López, Jonathan Rafael**
  **1006 Lausanne (CH)**
- **Richiardi, Jonas**
  **1202 Genève (CH)**
- **Kober, Tobias**
  **1025 St-Sulpice (CH)**
- **Maréchal, Bénédicte**
  **1040 Echallens (CH)**

(74) Representative: **Palaci, Ismaël**
  **Siemens Schweiz AG**
  **Intellectual Property**
  **Freilagerstrasse 40**
  **8047 Zürich (CH)**

(54) **SYSTEM AND METHOD FOR IMPROVING BIOLOGICAL OBJECT IMAGING**

(57) The invention relates to a system (200) and a method (100) for imaging a part of a biological object (210), the method (100) comprising:
- selecting (101) a reduced Image Acquisition Protocol, hereafter "IAP", for imaging said part of the biological object (210), wherein said reduced IAP has been obtained from a rich IAP by sub-sampling a set of imaging parameters of said rich IAP, wherein said set of imaging parameters are designed to control an acquisition of images by means of an imaging apparatus (220);
- using (102) said reduced IAP for acquiring one or several images of said part of the biological object (210);
- inputting (103) said one or several images into a trained Machine Learning, hereafter "ML", model that is configured for outputting, for each inputted image, one or several predicted images of said part of the biological object (210), wherein the ML model has been trained on input training data and output training data, wherein the input training data correspond to a first set of images obtained via said reduced IAP, and the output training data correspond to a corresponding second set of images obtained via said rich IAP;
- providing (104), via an interface (202), said one or several predicted images.

100

```
101 → 102 → 103 → 104
```

FIG 1

## Description

### Technical Field

The present invention is directed, in general, to biological imaging techniques used for imaging a biological object, like a brain. More specifically, the present invention is directed to methods and systems for improving image quality while decreasing its acquisition time.

### Background Art

Today, different biological imaging techniques exist for imaging a biological object. They are for instance Magnetic Resonance Imaging (MRI), Computed Tomography (CT), Positron Emission Tomography (PET), etc. Usually, a common problem faced by said biological imaging techniques is the acquisition time: the longer the acquisition time, the better the image quality. However, in real life, the duration of the acquisition time is limited due to clinical constraints. For instance, MRI has become an essential tool for clinical diagnostics, especially in emergency settings such as stroke management, where there is a critical need for fast and accurate diffusion-weighted MRI (DW-MRI) maps, including trace-weighted and Apparent Diffusion Coefficient (ADC) maps which measure the diffusivity of water in tissue, informing on pathological changes like edema and others. The imaging workup usually consists of several magnetic resonance (MR) contrast acquisitions wherein each of said acquisitions should be well under five minutes to allow rapid MR screening as required in current clinical guidelines. This is even more critical in emergency settings like in stroke, where "time is brain"; this is relevant as more and more hospitals introduce first-line MR imaging for stroke patients instead of the more common CT technique usually used for such cases. To meet these time requirements, MRI imaging protocols have been accelerated, featuring a reduced number of diffusion-encoding directions, lower spatial resolution, and rapid image processing techniques.

This approach, while time-efficient, introduces significant challenges in applications in computational imaging and machine learning. The primary issue is the low reproducibility and high inter-scan variability of the maps derived from these limited DWI acquisitions. The variability is often tissue-specific and can be subtle in many healthy brain regions. However, in clinical and Artificial Intelligence (AI)-based imaging applications, this variability undermines the reliability and usability of the diffusion-based MRI images and maps. A potential solution to improve image quality and reduce the said variability, for instance automated delineation of infarct core in acute ischemic stroke, is the use of high angular resolution diffusion imaging (HARDI) protocols. The latter proposes to increase the fidelity of DW-MRI by acquiring many diffusion gradient directions. While this approach allows for a more detailed reconstruction of the diffusion signal, thus improving the accuracy of the acquired images, the required acquisition of the numerous diffusion-encoding directions (typically more than 60) makes HARDI time-intensive and incompatible with the quick imaging timelines demanded in urgent clinical situations (as in stroke, for example).

Other techniques, like simultaneous multi-slice (SMS) acquisitions, compressed sensing and parallel imaging, have been proposed to reconstruct high-quality images from undersampled data. These methods leverage the redundancy of MR images to accelerate or parallelize (in the case of SMS) data acquisition, thus reducing scan time. However, they cannot fully compensate for the loss of information due to fewer gradient directions. Because of this, some research has focused on optimizing the diffusion gradient scheme to maximize the information obtained from a limited number of gradient directions and weightings. This includes strategic selection of gradient directions and strengths. These approaches are scanner-dependent, and cannot be universally applicable across different MRI systems, patient populations, or pathologies [1].

Other recent advancements have proposed to use AI and machine learning to improve image quality post-acquisition, reducing variability via harmonization techniques. This includes algorithms to adjust for scanner-specific biases or data quality transfer from low to high resolution protocols. The effectiveness of these AI models is however dependent on the diversity and quality of training data, or in acquiring same-subject paired data in multiple scanners, which is completely unfeasible in emergency-like settings [2].

For the currently existing biological imaging techniques, methods have thus been developed for mitigating the challenges associated with rapid and reliable imaging, but they usually come with their own set of limitations. There is therefore still a need for a novel approach that may improve image quality while being compatible with the quick imaging timelines that are required in urgent clinical situations.

### Summary of Invention

An objective of the present invention is to propose a method and a system for imaging a part of a biological object that overcome the above-mentioned issues.

Said objective is achieved according to the present invention by a method and a system according to the object of the independent claims. Dependent claims present further advantages of the invention.

The present invention concerns in particular a method for imaging a part of a biological object, said method comprising:

-    selecting a reduced Image Acquisition Protocol (IAP) for imaging said part of the biological object, wherein said reduced IAP has been obtained from a rich IAP by subsampling a set of imaging parameters of said rich IAP, wherein said set of imaging parameters are designed to control an acquisition of images by means of an imaging apparatus;
-    using said reduced IAP for acquiring one or several images of said part of the biological object, notably by means of said imaging apparatus, e.g. an MRI apparatus;
-    inputting said one or several images into a trained Machine Learning (ML) model or algorithm that is configured for outputting, for each inputted image, one or several predicted images of said part of the biological object, wherein the ML model has been trained on input training data and output training data, wherein the input training data correspond to a first set of images obtained via said reduced IAP, and the output training data correspond to a corresponding second set of images obtained via said rich IAP;
-    providing, via an interface, said one or several predicted images.

The present invention concerns also a system for imaging a part of a biological object by means of an imaging apparatus, said biological object being typically placed in an examination volume of said imaging apparatus, e.g. an MRI apparatus, the system comprising:

-    a control unit configured for controlling said imaging apparatus for acquiring images of said part of the biological object according to a reduced IAP, said control unit comprising a processor and a memory;
-    an interface, connected to the control unit, and configured for outputting predicted images;

said system being characterized in that its control unit is configured for carrying out the steps of the previously described method.

The present invention proposes also a computer-implemented method for providing a trained machine learning (ML) model or algorithm configured for outputting predicted images from one or several images of a part of a biological object, the method comprising:

-    acquiring at least one rich IAP, wherein each rich IAP is configured for defining a set of imaging parameters, and optionally sequences, designed for controlling an acquisition of images by means of an imaging apparatus;
-    for each rich IAP, sub-sampling said set of imaging parameters to compute a corresponding reduced IAP comprising a subset of said imaging parameters that, when implemented by said imaging apparatus, enables the latter to perform an image acquisition by executing said reduced IAP, and storing for each rich IAP its corresponding reduced IAP;
-    for each training biological object of a group of training biological objects, and for each rich IAP, using its corresponding reduced IAP for acquiring a first set of images of said part of the training biological object, and using said rich IAP for acquiring a corresponding second set of images, called hereafter ground truth images, of said part of the training biological object, each first set of images being therefore related to a corresponding second set of images;
-    using at least one of said first sets of images and its respective corresponding second set of images for training an ML model, wherein for said training, the ML model receives said first set of images as input training data, its respective corresponding second set of images as output training data. Preferentially, some or all first sets of images and their respective corresponding second sets are used for said training;
-    training the ML model based on the input training data and output training data;
-    providing the trained ML model with an output interface.

**Description of Embodiments**

The present invention proposes to use a reduced IAP for acquiring images of the biological object (i.e. a first set of images) instead of a corresponding rich IAP, wherein said reduced IAP is configured for reducing the image acquisition time by using a subsample of the imaging parameters of the rich IAP, while obtaining at the end, and thanks to said trained ML model, a set of images comprising additional images compared to said first set (i.e. an augmented set of images corresponding to the first set of images augmented with predicted images) that is at least equivalent to a set of images that would have been obtained when using the rich IAP. In order to subsample the imaging parameters of said rich IAP, the present method uses preferentially a subsampling rule that might be stored in a memory of the system according to the invention. In particular, the present invention may define, for each image acquisition technique, like DW MRI, an associated subsampling rule configured for technically characterizing the subsampling of the set of imaging parameters of the rich IAP. Thanks to this subsampling coupled to the ML training method, the present invention enables to obtain, for the reduced IAP, a same quality of "derived maps" as would have been obtained from the corresponding rich IAP (e.g. maps obtained by applying a segmentation method to a reduced IAP are of the same quality as maps obtained by applying said segmentation method to the corresponding rich IAP), while reducing the acquisition time. The subsampling rule enables to select a representative subset of the imaging parameters of the rich IAP, e.g. a representative subset of diffusion gradient directions, which ensures that essential information is captured for computing an image, despite using fewer imaging acquisition parameters.

According to the present invention, the rich IAP is an imaging protocol that defines a set of imaging parameters designed for controlling an acquisition of images by an imaging apparatus usually according to an imaging sequence based on said imaging parameters, wherein the number of said imaging parameters in said rich IAP set exceeds a minimum number of said imaging parameters that would be required or necessary for encoding a minimum amount of signal information for a computation of signal-based metrics. Also, said minimum amount of signal information would not be sufficient, i.e. would be too poor, for computing images useful in clinical applications. Typically, a rich IAP depends on the imaging technique. For instance, in DW-MRI, a rich IAP is a high-angular resolution protocol with a large number of 3D gradient directions (b-vectors) at each b-value, alongside a dense sampling of gradient field strength and diffusion gradient timings, as indicated by the b-value equation: $b = \gamma^2 \cdot G^2 \cdot \delta^2 \cdot (\Delta - \delta/3)$, wherein $\gamma$ is the gyromagnetic ratio, G is the gradient pulse magnitude, $\delta$ its duration, $\Delta$ the time interval separating two directly neighboring gradient pulses. In multi-echo T2 relaxometry MRI, a rich IAP is for instance a protocol that comprises a large number of echo times, surpassing the minimum required number to accurately characterize the T2 relaxation curve. Similarly, in Perfusion-Weighted MRI (PW-MRI), which includes techniques like Dynamic Susceptibility Contrast (DSC) and Arterial Spin Labeling (ASL), a rich IAP is a protocol configured for acquiring a high number of time points, also known as time frames, to comprehensively capture the dynamic processes. In each case, the present invention proposes to compute a reduced IAP by subsampling the set of imaging parameters, for instance, by subsampling the set of b-values and/or b-vectors in DW-MRI, by subsampling the set of echo times in T2 relaxometry, and by subsampling the set of time points in PW-MRI.

Advantageously, the present invention applies to different imaging techniques. As demonstrated above, the concept might be applied to MRI techniques used for imaging said part of the biological object, but also to CT imaging techniques, or to PET imaging techniques. Preferentially, said rich IAP is for instance an MRI DW acquisition protocol, wherein said imaging parameters comprise diffusion encoding directions (i.e. the so-called b-vectors or gradient directions), and b-values for DW image acquisition. Typically, the MRI DW rich IAP comprises more than 60 gradient directions. It is for instance a HARDI protocol. Said MRI DW acquisition protocol typically further comprises at least one an MRI acquisition sequence designed for DW image acquisition based on said imaging parameters. In this case, the corresponding reduced IAP is an angular-reduced acquisition protocol obtained by subsampling the diffusion encoding directions of said MRI rich IAP and/or said b-values. In particular, the subsampling rule might define, notably during the training of the ML model, that the number of sub-sampled diffusion encoding directions is fixed to a given number of gradient directions, or that the subsampling is determined by computing, from said diffusion encoding directions, an optimal equispaced sub-set of gradient directions of size N.

In case of dynamic CT imaging, said rich IAP is a protocol defining a set of acquisition parameters comprising a large number of projections (e.g. of X-ray scattering angles) and time points. Then, the reduced IAP according to the invention comprises a subsample of said projections and/or time points. In case of PET, said rich IAP is for instance a protocol defining a set of acquisition time series comprising a number of acquisition time series that is sufficient for capturing the temporal dynamics for a considered PET application. For instance, said number might be at least twice as high as the minimum number of said acquisition time series required for capturing said temporal dynamics. In such a case, the reduced IAP comprises a subsample or subset of said acquisition time series, wherein the number of the latter is greater than or equal to said minimum number required to capture said temporal dynamics, but smaller than the full set of time points originally defined in the rich IAP.

The present invention proposes in particular to use data augmentation and/or contrastive learning for training said ML model and improve its output. Preferentially, said contrastive learning comprises using a similarity measure for quantifying a similarity between (i) predicted images obtained for a biological object from the trained ML model and (ii) ground truth images obtained for the same biological object, and using said similarity measure for further training the trained ML model. Preferably, said similarity measure uses a Dice coefficient. The present invention also proposes to use a sigmoid penalty function for training the ML model. Preferably, the latter is calculated in function of a variability of said similarity measure when considering different predicted images for a same ground truth image.

Preferably, for training said ML model, said second set of images is acquired for each rich IAP, and, for each rich IAP, several sets of images matching the reduced IAP (i.e. satisfying the subsampling rule) might be used for training the ML model. Indeed, for a same reduced IAP, different subsampling of the imaging parameters of the rich IAP may take place, each satisfying the subsampling rule, giving rise to different "first sets" of images for said same reduced IAP, wherein said different first sets are not identical. For instance, the subset of imaging parameters may comprise a same number of gradient directions (e.g. 3 from 64), but not the same gradient directions. This gives rise to different first sets of images that satisfy the subsampling technique/rule defined for the reduced IAP (e.g. the sub-sampling technique can be fixed to a given number of gradient directions, or by computing the optimal equispaced sub-set of size n). Therefore, for a same ground truth image, different predicted maps or images ($P\_1, ... ,P\_n$) might be obtained by applying different subsampling that match the subsampling rule defined for the reduced IAP, said predicted maps being then compared to the ground truth image for improving the ML model.

**Brief Description of the Drawing**

For a more complete understanding of the present disclosure, and the advantages thereof, reference is now made to the following descriptions taken in conjunction with the accompanying drawings, wherein like numbers designate like objects, and in which:

Fig. 1 illustrates a flowchart of a method for according to the invention;
Fig. 2 illustrates a preferred embodiment of a system according to the invention.

**Description of Examples**

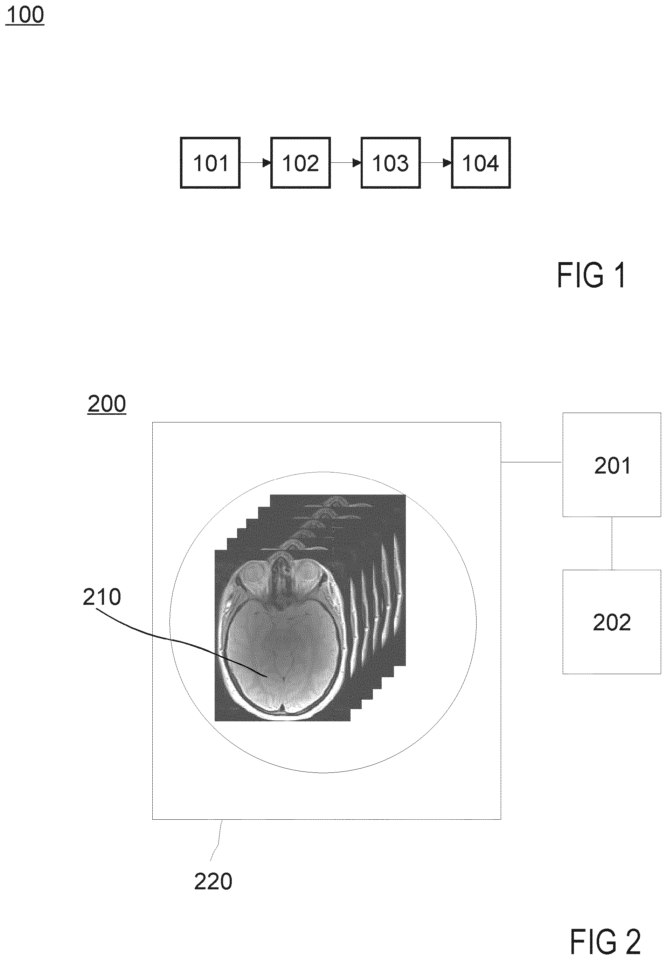

We will now describe in more detail a preferred embodiment of the method according to the invention through Figures 1 and 2, wherein Figure 1 describes the different steps of the method 100 carried out by a preferred embodiment of a system 200 according to the invention illustrated by Figure 2.

The system 200 according to the invention comprises a control unit 201 configured for controlling an imaging apparatus 220, for instance an MRI apparatus, for imaging a part of a biological object 210, e.g. a brain, which is typically placed in an examination volume of the imaging apparatus 220. The system 200 further comprises an interface 202 connected to the control unit 201 for outputting predicted images computed by the control unit 201. The later typically comprises a processor and a memory, wherein said processor is configured for running a trained ML model that computes said predicted images. Said interface 202 may comprise a display for displaying said predicted images. The system 200 is configured for carrying out the following steps of the method:

At step 101, the system 200 automatically selects a reduced IAP for imaging said part of the biological object 210. For instance, in function of an imaging technique selected by a user, the system automatically determines, among a set of reduced IAPs, which one has to be used. Alternatively, a user or operator may select the reduced IAP that has to be used for imaging the biological object. The reduced IAP comprises a subset of imaging parameters of a rich IAP, and is configured for controlling an acquisition of images by the imaging apparatus 220. Said imaging parameters are typically acquisition parameters that define or specify how images will be collected/acquired by the imaging apparatus, and thus which actions have to be performed by the imaging apparatus for collecting/acquiring said images. In other words, the different image acquisition steps performed by the imaging apparatus for acquiring said images highly depend on said imaging parameters, and thus the duration of the acquisition will also depend on said imaging parameters.

At step 102, the system 200 controls the imaging apparatus 220 according to the reduced IAP for acquiring one or several images of said part of the biological object 210.

At step 103, the system 200 uses said one or several images as input to the trained ML model that outputs, for each inputted image, one or several predicted images of said part of the biological object 210. Preferentially, a data augmentation technique based on the obtained predicted images is used for enhancing the training of the ML model during a training phase of said ML model.

At step 104, the system 200 provides said one or several predicted images via the interface 202. For instance, the predicted images might be displayed on a screen for visualization.

For subsampling a set of imaging parameters of a rich IAP, the present invention proposes to use a subsampling rule. Typically, for each imaging technique, the system may store a subsampling rule in a memory, which, when applied to a rich IAP, may automatically subsample the rich IAP set of imaging parameters for computing the reduced IAP. For instance, for DW MRI, the subsampling rule may require subsampling the diffusion encoding directions (b-vectors) to compute a reduced IAP that is, in this case, an angular-reduced acquisition protocol, from which the obtained predicted images are "derivative diffusion images". Alternatively, or additionally, the system may store for each imaging technique a corresponding reduced IAP.

The present invention further proposes to improve the robustness of the ML model by automatically computing a variability of a similarity measure (e.g., the Dice coefficient that quantifies overlap) between the predicted and ground truth images, and using said variability for improving the training of the ML model. This enables notably to assess a dependency of the ML model on subsampling changes, given that for a same subsampling rule, different subsets of the rich IAP imaging parameters might be computed.

Said similarity measure is configured for quantifying the similarity between a predicted image and a corresponding ground truth image. The corresponding ground truth image is an image representing, with respect to said biological object, a same biological object region as the predicted image. Indeed, the ML model outputs, from a first set of images, a second set of images that are predicted images, wherein each image of said second set corresponds to an image that would have been computed if the rich IAP would have been performed. In particular, the Dice coefficient might be used for computing said similarity measure. It is calculated for each pair of predicted and ground truth images according to the equation:

$$Dice(P, GT) = \frac{2 \times |P \cap GT|}{|P| + |GT|} \qquad \text{Eq. 1}$$

where P and GT are the predicted and ground truth binary images, respectively. In particular, the present invention proposes to gauge the ML model across different predicted image datasets that are obtained via different subsampling of a same rich IAP set of imaging parameters by calculating a variability of these Dice scores according to the following equation:

$$Variability = Var(Dice(P\_1, GT), Dice(P\_2, GT), \dots, Dice(P\_n, GT)) \qquad \text{Eq. 2}$$

where $(P\_1, P\_2, ..., P\_n)$ are the different predicted maps obtained for the same ground truth image GT. Preferentially, the Dice coefficient term might be replaced by a given similarity measurement function. Optionally, the variability might also be computed as a combination of different similarity scores computed for each pair of predictive/ground truth images.

Preferentially, during said training of the ML model, the system is further configured for computing a sigmoid penalty in function of said variability according to:

$$Penalty(Variability) = (1 + e^{-k(Variability - \theta)})^{-1} \quad \text{Eq. 3}$$

where k controls a steepness of the sigmoid and $\theta$ sets the midpoint of said sigmoid.

During the training, the computed penalty function is configured for increasing a cost in a backpropagation step proportionally to the variability in a controlled, non-linear fashion. The backpropagation step is configured for optimizing weights of the ML model (e.g. the ML model is a feedforward neural network and the backpropagation step optimizes weights of said neural network) so that it learns to correctly map its input training data to its output training data, the difference between predicted output data and the output training data being measured by said cost. According to the present invention, said cost will notably be augmented by either adding the variability penalty (Eq. 3), or by using Eq. 3 to modulate a step size on a gradient descent method during the backpropagation step. The sigmoidal nature of the penalty ensures a balanced response to variability, applying a moderate penalty for slight deviations in the similarity scores, intensifying as variability increases, and then plateauing at high variability levels. Thanks to this method, the ML model might be reinforced where consistency was lacking, and additionally, it finely tunes the model's learning process to nuances of the data it encounters. The sigmoid penalty function, based on the variability of one or more similarity scores and sub-sampled predicted images (e.g. DW MRI diffusion images), enables thus to adjust the training of the ML model for ensuring that image regions with higher variability due to constraints of the reduced IAP are prioritized, leading to a more balanced and effective learning process. The method according to the invention addresses the inherent variability in imaging biological tissues, where the interaction between the imaging protocol and tissue characteristics often results in regions with higher noise, variability, or artifacts. By training the model with augmented and simulated data that replicates these complexities, the claimed method ensures that regions affected by the combined influence of tissue properties and the constraints of the reduced acquisition protocol (AIP) are better managed. In particular, the model dynamically adjusts its learning rate in response to these areas of increased variability, enabling more precise learning and improved domain adaptation. As a result, the model becomes more effective at handling both the imaging data and the variability introduced by said biological objects.

Advantageously, the present concept allows to develop reduced IAPs that fit within specific image acquisition time and/or hardware constraints by defining subsampling rules constraining the reduced IAP to fit within said specific image acquisition time and/or hardware constraint. The present concept ensures that, while rich IAP (i.e. high-resolution protocols) address the intricacies and variability inherent in detailed images, the corresponding reduced IAP (i.e. lowresolution protocols) can still be integrated effectively into the ML model training. This integration not only enhances model robustness but also ensures adaptability across a range of imaging conditions. In essence, the method facilitates the creation of a spectrum of protocols, from high to low resolution, each tailored for optimal performance under different clinical and operational constraints.

According to the present invention, the same ML model might be used for different IAP. In particular, the present invention proposes to generate synthetic images (i.e. said predicted images), e.g. synthetic diffusion data, with different reduced IAPs, and to use the generated synthetic data for data augmentation for training the ML model. In particular, the predicted images can be used to add a different contrastive learning term to the ML model, which can improve robustness to changes in IAP by forcing a neural network to learn internal representations which are similar for different IAP, e.g. for different diffusion protocols in MRI.

Advantageously, for computing the reduced IAP, the system preferably reuses image acquisition routine data that can be obtained directly from existing rich IAPs, e.g. as it exists in 1.5T and 3T MRI image acquisition settings. This approach allows for improving the robustness of the ML model by accounting for protocol changes without the time-intensive demands of HARDI, making the present invention suitable for urgent medical situations where quick imaging is paramount. In addition, by using a subsampling strategy that addresses rich IAP variability (i.e. through the calculation of said variability), the ML model becomes effectively trained and validated across a wide range of scenarios, including emergency settings that might be used in concrete clinical applications.

To conclude, through the computing and use of reduced IAPs for imaging a biological object, the present invention allows for high-quality imaging without necessitating an extensive amount of imaging parameters (e.g. an extensive number of gradient directions), thereby speeding up the imaging process and reducing operational time. This invention is particularly well-suited to DWMRI.

**List of citations**

[1] Alexander DC and Barker GJ, Optimal imaging parameters for fiber-orientation estimation in diffusion MRI. Neuroimage 27(2):357-67, 2005.

[2] Mirzaalian H, et al., Inter-site and inter-scanner diffusion MRI data harmonization. Neuroimage 135: 311-23, 2016

**Claims**

1. Method (100) for imaging a part of a biological object (210), the method (100) comprising:

   - selecting (101) a reduced Image Acquisition Protocol, hereafter "IAP", for imaging said part of the biological object (210), wherein said reduced IAP has been obtained from a rich IAP by sub-sampling a set of imaging parameters of said rich IAP, wherein said set of imaging parameters are designed to control an acquisition of images by means of an imaging apparatus (220);
   - using (102) said reduced IAP for acquiring one or several images of said part of the biological object (210);
   - inputting (103) said one or several images into a trained Machine Learning, hereafter "ML", model that is configured for outputting, for each inputted image, one or several predicted images of said part of the biological object (210), wherein the ML model has been trained on input training data and output training data, wherein the input training data correspond to a first set of images obtained via said reduced IAP, and the output training data correspond to a corresponding second set of images obtained via said rich IAP;
   - providing (104), via an interface (202), said one or several predicted images.

2. Method (100) according to claim 1, comprising:

   a) using a Magnetic Resonance Imaging, hereafter "MRI", technique for imaging said part of the biological object (210), said rich IAP being an MRI rich IAP ; or
   b) using a Computed Tomography, hereafter "CT", imaging technique for imaging said part of the biological object (210), said rich IAP being a CT rich IAP; or
   c) using a Positron Emission Tomography, hereafter "PET", imaging technique for imaging said part of the biological object (210), said rich IAP being a PET rich IAP.

3. Method (100) according to claim 2, wherein for step (a), said MRI rich IAP is an MRI diffusion-weighted, hereafter DW, acquisition protocol, wherein said imaging parameters comprise diffusion encoding directions, i.e. gradient directions, and b-values for DW image acquisition, said rich IAP further comprising at least one an MRI sequence designed for DW image acquisition based on said imaging parameters, and wherein said reduced IAP is an angular-reduced acquisition protocol obtained by sub-sampling the diffusion encoding directions of said MRI rich IAP and/or said b-values.

4. Method according to claim 2, wherein, for step (b), said CT rich IAP is a protocol defining a set of acquisition parameters comprising a large number of projections and time points, and said reduced IAP comprises a subsample of said projections and/or time points.

5. Method (100) according to claim 3, wherein the number of sub-sampled diffusion encoding directions is fixed to a given number of gradient directions, or is determined by computing, from said diffusion encoding directions, an optimal equispaced sub-set of gradient directions of size N.

6. Method (100) according to one of the claims 1 to 5, comprising using data augmentation and/or contrastive learning for training said ML model.

7. Computer-implemented method for providing a trained Machine Learning, hereafter "ML", model configured for outputting predicted images from one or several images of a part of a biological object (220), the method comprising:

- acquiring at least one rich Image Acquisition Protocol, hereafter "IAP", wherein each rich IAP is configured for defining a set of imaging parameters designed for controlling an acquisition of images by means of an imaging apparatus (220) ;
- for each rich IAP, sub-sampling said set of imaging parameters to compute a corresponding reduced IAP comprising a subset of said imaging parameters that, when implemented by said imaging apparatus (220), enables the latter to perform an image acquisition by executing said reduced IAP, and storing for each rich IAP its corresponding reduced IAP;
- for each training biological object (210) of a group of training biological objects (210), and for each rich IAP, using its corresponding reduced IAP for acquiring a first set of images of said part of the training biological object (210), and using said rich IAP for acquiring a corresponding second set of images (210), called hereafter ground truth images, of said part of the training biological object (210), each first set of images being therefore related to a corresponding second set of images;
- using at least one of said first sets of images and its corresponding second set of images for training an ML model, wherein for said training, the ML model receives said first set of images as input training data and the corresponding second set of images as output training data;
- training the ML model based on the input training data and output training data;
- providing the trained ML model with an output interface (202).

8. Computer-implemented method according to claim 7, wherein said rich IAP is:

a) a Magnetic Resonance Imaging, hereafter "MRI", IAP configured for imaging said part of the biological object (210), said rich IAP being an MRI rich IAP; or
b) a Computed Tomography, hereafter "CT", imaging technique configured for imaging said part of the biological object (210), said rich IAP being a CT rich IAP; or
c) a Positron Emission Tomography, hereafter "PET", imaging technique configured for imaging said part of the biological object (210), said rich IAP being a PET rich IAP.

9. Computer-implemented method according to claim 8, wherein, for step (a), said rich MRI IAP is an MRI diffusion-weighted, hereafter DW, acquisition protocol, wherein said imaging parameters comprise diffusion encoding directions, i.e. gradient directions, and b-values for DW image acquisition, wherein said rich IAP further comprises at least one an MRI sequence designed for DW image acquisition based on said imaging parameters, and wherein said reduced IAP is an angular-reduced acquisition protocol obtained by sub-sampling the diffusion encoding directions of said MRI rich acquisition protocol and/or said b-values.

10. Computer-implemented method according to claim 9, wherein the number of sub-sampled diffusion encoding directions is fixed and equal to a given number of gradient directions, or is determined by computing, from said diffusion encoding directions, an optimal equispaced sub-set of gradient directions of size N.

11. Computer-implemented method according to one of the claims 7 to 10, comprising using data augmentation and/or contrastive learning for training said ML model.

12. Computer-implemented method according to claim 11, wherein said contrastive learning comprises using a similarity measure for quantifying a similarity between (i) a predicted image obtained for one of said training biological objects from the trained ML model and (ii) a corresponding ground truth image obtained for the same training biological object, and using said similarity measure for further training the trained ML model.

13. Computer-implemented method according to claim 12, wherein said similarity measure uses a Dice coefficient.

14. Computer-implemented method according to one of the claims 7-13, comprising using a sigmoid penalty function for training the ML model.

15. System (200) for imaging a part of a biological object (210) placed in an examination volume (221) of an imaging apparatus (220), the system (200) comprising:

    - a control unit (201) configured for controlling said imaging apparatus (220) for acquiring images of said part of the biological object according to a reduced image acquisition protocol, hereafter IAP, said control unit comprising a processor and a memory;
    - an interface (202), connected to the control unit (201), and configured for outputting predicted images;

said system (200) being **characterized in that** its control unit (201) is configured for carrying out the steps of the method (100) according to one of the claims 1 to 6.

100

101 → 102 → 103 → 104

FIG 1

200

210

220

FIG 2

Europäisches
Patentamt

European
Patent Office

Office européen
des brevets

## PARTIAL EUROPEAN SEARCH REPORT

under Rule 62a and/or 63 of the European Patent Convention.
This report shall be considered, for the purposes of
subsequent proceedings, as the European search report

**Application Number**

EP 24 20 3534

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | YOON YEO HUN ET AL: "Efficient B-Mode Ultrasound Image Reconstruction From Sub-Sampled RF Data Using Deep Learning", IEEE TRANSACTIONS ON MEDICAL IMAGING, IEEE, USA, vol. 38, no. 2, 1 February 2019 (2019-02-01), pages 325-336, XP011707491, ISSN: 0278-0062, DOI: 10.1109/TMI.2018.2864821 [retrieved on 2019-01-31] * page 326 - page 333; figures 1, 3, 5, 6, 10 * ----- -/-- | 1,15 | INV. G01R33/56 G01R33/561 ADD. A61B6/03 G01R33/563 |

**TECHNICAL FIELDS SEARCHED (IPC)**

G01R
A61B

## INCOMPLETE SEARCH

The Search Division considers that the present application, or one or more of its claims, does/do not comply with the EPC so that only a partial search (R.62a, 63) has been carried out.

Claims searched completely :

Claims searched incompletely :

Claims not searched :

Reason for the limitation of the search:

**see sheet C**

| Place of search | Date of completion of the search | Examiner |
|---|---|---|
| Munich | 27 June 2025 | Raguin, Guy |

Europäisches
Patentamt

European
Patent Office

Office européen
des brevets

**PARTIAL EUROPEAN SEARCH REPORT**

**Application Number**

EP 24 20 3534

| | DOCUMENTS CONSIDERED TO BE RELEVANT | | CLASSIFICATION OF THE APPLICATION (IPC) |
|---|---|---|---|
| Category | Citation of document with indication, where appropriate, of relevant passages | Relevant to claim | |
| X | RASTI PEJMAN ET AL: "Convolutional Neural Network Super Resolution for Face Recognition in Surveillance Monitoring", 2 July 2016 (2016-07-02), SAT 2015 18TH INTERNATIONAL CONFERENCE, AUSTIN, TX, USA, SEPTEMBER 24-27, 2015; [LECTURE NOTES IN COMPUTER SCIENCE; LECT.NOTES COMPUTER], SPRINGER, BERLIN, HEIDELBERG, PAGE(S) 175 - 184, XP047350321, ISBN: 978-3-540-74549-5 [retrieved on 2016-07-02] * page 177 - page 182 * ----- | 1,15 | |
| X | US 2019/172230 A1 (MAILHE BORIS [US] ET AL) 6 June 2019 (2019-06-06) * paragraphs [0004] - [0007], [0040], [0042] - paragraph [0050]; figures 1, 2 * ----- | 1,2,15 | **TECHNICAL FIELDS SEARCHED** (IPC) |
| X | US 2019/266761 A1 (MALKIEL ITZIK [IL] ET AL) 29 August 2019 (2019-08-29) * paragraph [0016] - paragraph [0022]; figures 1, 2 * ----- | 1,2,15 | |
| X | GOLKOV VLADIMIR ET AL: "q-Space Deep Learning: Twelve-Fold Shorter and Model-Free Diffusion MRI Scans", IEEE TRANSACTIONS ON MEDICAL IMAGING, IEEE, USA, vol. 35, no. 5, 1 May 2016 (2016-05-01), pages 1344-1351, XP011607959, ISSN: 0278-0062, DOI: 10.1109/TMI.2016.2551324 [retrieved on 2016-04-29] * page 1345 - page 1349; figure 1 * ----- | 1-3,5,15 | |

-/--

EPO FORM 1503 03.82 (P04C10)

Europäisches
Patentamt

European
Patent Office

Office européen
des brevets

## PARTIAL EUROPEAN SEARCH REPORT

**Application Number**

EP 24 20 3534

| | DOCUMENTS CONSIDERED TO BE RELEVANT | | CLASSIFICATION OF THE APPLICATION (IPC) |
|---|---|---|---|
| Category | Citation of document with indication, where appropriate, of relevant passages | Relevant to claim | |
| L | GOLKOV VLADIMIR ET AL: "q-Space Deep Learning for Twelve-Fold Shorter and Model-Free Diffusion MRI Scans", 18 November 2015 (2015-11-18), SAT 2015 18TH INTERNATIONAL CONFERENCE, AUSTIN, TX, USA, SEPTEMBER 24-27, 2015; [LECTURE NOTES IN COMPUTER SCIENCE; LECT.NOTES COMPUTER], SPRINGER, BERLIN, HEIDELBERG, PAGE(S) 37 - 44, XP047326268, ISBN: 978-3-540-74549-5 [retrieved on 2015-11-18] * page 38 - page 42; figures 2, 3 * ----- | 1-3,5,15 | |
| | | | **TECHNICAL FIELDS SEARCHED (IPC)** |

EPO FORM 1503 03.82 (P04C10)

page 3 of 3

Europäisches
Patentamt

European
Patent Office

Office européen
des brevets

**INCOMPLETE SEARCH
SHEET C**

Application Number

EP 24 20 3534

Claim(s) completely searchable:
        1-6, 15

Claim(s) not searched:
        7-14

Reason for the limitation of the search:

Pursuant to Rule 62a(1) EPC, the applicant was invited in the
communication dated 02-02-2025 to indicate one of independent method
claims 1 and 7, on the basis of which the search is to be carried out.

In their response dated 01-04-2025, the applicant requested that the
search be carried out on the basis of (at least) independent claim 1.
They further argued that claims 1 and 7 are based on the same concept.
However, this line of argument corresponds to arguments against a lack of
unity objection (Art. 82 EPC), and cannot be considered convincing
against the present objection pursuant Rule 43(2) EPC, since the
inter-relation requirement of Rule 43(2) EPC argued in the communication
dated 02-02-2025 is more stringent than merely being based on the same
concept.

Therefore, claim 7 and any reference thereto are excluded from the
present search, such that the search is carried out on the basis of
claims 1-6 and 15.

Europäisches
Patentamt

European
Patent Office

Office européen
des brevets

---

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

---

## LACK OF UNITY OF INVENTION

The Search Division considers that the present European patent application does not comply with the requirements of unity of invention and relates to several inventions or groups of inventions, namely:

    see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☒ Only part of the further search fees have been paid within the fixed time limit. The present European search report has been drawn up for those parts of the European patent application which relate to the inventions in respect of which search fees have been paid, namely claims:

    1, 3, 5(completely); 2, 15(partially)

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION**
**SHEET B**

**Application Number**

**EP 24 20 3534**

The Search Division considers that the present European patent application does not comply with the requirements of unity of invention and relates to several inventions or groups of inventions, namely:

1. claims: 1(completely); 2, 15(partially)

Method and corresponding system for imaging a part of a biological object, comprising: selecting a reduced Image Acquisition Protocol, hereafter "IAP", for imaging said part of the biological object, wherein said reduced IAP has been obtained from a rich IAP by sub-sampling a set of imaging parameters of said rich IAP, wherein said set of imaging parameters are designed to control an acquisition of images by means of an imaging apparatus; using said reduced IAP for acquiring one or several images of said part of the biological object; inputting said one or several images into a trained Machine Learning, hereafter "ML", model that is configured for outputting, for each inputted image, one [...] predicted image[...] of said part of the biological object, wherein the ML model has been trained on input training data and output training data, wherein the input training data correspond to a first set of images obtained via said reduced IAP, and the output training data correspond to a corresponding second set of images obtained via said rich IAP; providing, via an interface, said one or several predicted images; said method and system further using a Magnetic Resonance Imaging, hereafter "MRI", technique for imaging said part of the biological object, said rich IAP being an MRI rich IAP.

---

2. claims: 4(completely); 2, 6, 15(partially)

Method and corresponding system for imaging a part of a biological object, comprising: selecting a reduced Image Acquisition Protocol, hereafter "IAP", for imaging said part of the biological object, wherein said reduced IAP has been obtained from a rich IAP by sub-sampling a set of imaging parameters of said rich IAP, wherein said set of imaging parameters are designed to control an acquisition of images by means of an imaging apparatus; using said reduced IAP for acquiring one or several images of said part of the biological object; inputting said one or several images into a trained Machine Learning, hereafter "ML", model that is configured for outputting, for each inputted image, one [...] predicted image[...] of said part of the biological object, wherein the ML model has been trained on input training data and output training data, wherein the input training data correspond to a first set of images obtained via said reduced IAP, and the output training data correspond to a corresponding second set of images obtained via said rich IAP; providing, via an interface, said one or several predicted images; said method and system further using a Computed Tomography, hereafter "CT", technique for imaging said part of the biological object, said rich IAP

Europäisches
Patentamt

European
Patent Office

Office européen
des brevets

**LACK OF UNITY OF INVENTION**
**SHEET B**

Application Number

EP 24 20 3534

The Search Division considers that the present European patent application does not comply with the requirements of unity of invention and relates to several inventions or groups of inventions, namely:

being a CT rich IAP.

---

3. claims: 2, 6, 15(all partially)

Method and corresponding system for imaging a part of a biological object, comprising: selecting a reduced Image Acquisition Protocol, hereafter "IAP", for imaging said part of the biological object, wherein said reduced IAP has been obtained from a rich IAP by sub-sampling a set of imaging parameters of said rich IAP, wherein said set of imaging parameters are designed to control an acquisition of images by means of an imaging apparatus; using said reduced IAP for acquiring one or several images of said part of the biological object; inputting said one or several images into a trained Machine Learning, hereafter "ML", model that is configured for outputting, for each inputted image, one [...] predicted image[...] of said part of the biological object, wherein the ML model has been trained on input training data and output training data, wherein the input training data correspond to a first set of images obtained via said reduced IAP, and the output training data correspond to a corresponding second set of images obtained via said rich IAP; providing, via an interface, said one or several predicted images; said method and system further using a Positron Emission Tomography, hereafter "PET", technique for imaging said part of the biological object, said rich IAP being a PET rich IAP.

---

4. claims: 3, 5(completely); 15(partially)

Method and corresponding system for imaging a part of a biological object, comprising: selecting a reduced Image Acquisition Protocol, hereafter "IAP", for imaging said part of the biological object, wherein said reduced IAP has been obtained from a rich IAP by sub-sampling a set of imaging parameters of said rich IAP, wherein said set of imaging parameters are designed to control an acquisition of images by means of an imaging apparatus; using said reduced IAP for acquiring one or several images of said part of the biological object; inputting said one or several images into a trained Machine Learning, hereafter "ML", model that is configured for outputting, for each inputted image, [...] several predicted images of said part of the biological object, wherein the ML model has been trained on input training data and output training data, wherein the input training data correspond to a first set of images obtained via said reduced IAP, and the output training data correspond to a corresponding second set of images obtained via said rich IAP; providing, via an interface, said one or

page 2 of 4

Europäisches
Patentamt

European
Patent Office

Office européen
des brevets

**LACK OF UNITY OF INVENTION
SHEET B**

**Application Number**

EP 24 20 3534

The Search Division considers that the present European patent application does not comply with the requirements of unity of invention and relates to several inventions or groups of inventions, namely:

several predicted images; said method and system further comprising using a Magnetic Resonance Imaging, hereafter "MRI", technique for imaging said part of the biological object, said rich IAP being an MRI rich IAP; wherein said MRI rich IAP is an MRI diffusion-weighted, hereafter DW, acquisition protocol, wherein said imaging parameters comprise diffusion encoding directions, i.e. gradient directions, and b-values for DW image acquisition, said rich IAP further comprising at least one an MRI sequence designed for DW image acquisition based on said imaging parameters, and wherein said reduced IAP is an angular-reduced acquisition protocol obtained by sub-sampling the diffusion encoding directions of said MRI rich IAP and/or said b-values.

---

5. claims: 6, 15(all partially)

Method and corresponding system for imaging a part of a biological object, comprising: selecting a reduced Image Acquisition Protocol, hereafter "IAP", for imaging said part of the biological object, wherein said reduced IAP has been obtained from a rich IAP by sub-sampling a set of imaging parameters of said rich IAP, wherein said set of imaging parameters are designed to control an acquisition of images by means of an imaging apparatus; using said reduced IAP for acquiring one or several images of said part of the biological object; inputting said one or several images into a trained Machine Learning, hereafter "ML", model that is configured for outputting, for each inputted image, one or several predicted images of said part of the biological object, wherein the ML model has been trained on input training data and output training data, wherein the input training data correspond to a first set of images obtained via said reduced IAP, and the output training data correspond to a corresponding second set of images obtained via said rich IAP; providing, via an interface, said one or several predicted images; wherein said method and system further comprises using data augmentation for training said ML model.

---

6. claims: 6, 15(all partially)

Method and corresponding system for imaging a part of a biological object, comprising: selecting a reduced Image Acquisition Protocol, hereafter "IAP", for imaging said part of the biological object, wherein said reduced IAP has been obtained from a rich IAP by sub-sampling a set of imaging parameters of said rich IAP, wherein said set of imaging parameters are designed to control an acquisition of images

Europäisches
Patentamt

European
Patent Office

Office européen
des brevets

**LACK OF UNITY OF INVENTION
SHEET B**

The Search Division considers that the present European patent application does not comply with the requirements of unity of invention and relates to several inventions or groups of inventions, namely:

by means of an imaging apparatus; using said reduced IAP for acquiring one or several images of said part of the biological object; inputting said one or several images into a trained Machine Learning, hereafter "ML", model that is configured for outputting, for each inputted image, one or several predicted images of said part of the biological object, wherein the ML model has been trained on input training data and output training data, wherein the input training data correspond to a first set of images obtained via said reduced IAP, and the output training data correspond to a corresponding second set of images obtained via said rich IAP; providing, via an interface, said one or several predicted images; wherein said method and system further comprises using contrastive learning for training said ML model.

---

page 4 of 4

## ANNEX TO THE EUROPEAN SEARCH REPORT
## ON EUROPEAN PATENT APPLICATION NO.

EP 24 20 3534

This annex lists the patent family members relating to the patent documents cited in the above-mentioned European search report.
The members are as contained in the European Patent Office EDP file on
The European Patent Office is in no way liable for these particulars which are merely given for the purpose of information.

27-06-2025

| Patent document cited in search report | Publication date | Patent family member(s) | | Publication date |
|---|---|---|---|---|
| US 2019172230 A1 | 06-06-2019 | NONE | | |
| US 2019266761 A1 | 29-08-2019 | CN | 110211050 A | 06-09-2019 |
| | | EP | 3534170 A1 | 04-09-2019 |
| | | US | 2019266761 A1 | 29-08-2019 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ALEXANDER DC** ; **BARKER GJ**. Optimal imaging parameters for fiber-orientation estimation in diffusion MRI. *Neuroimage*, 2005, vol. 27 (2), 357-67

- **MIRZAALIAN H et al.** Inter-site and inter-scanner diffusion MRI data harmonization. *Neuroimage*, 2016, vol. 135, 311-23